Europäisches Patentamt

European Patent Office

Office européen des brevets

(11) Publication number: **0 087 869**
**B1**

(12) **EUROPEAN PATENT SPECIFICATION**

(45) Date of publication of patent specification: **16.07.86**

(21) Application number: **83300661.2**

(22) Date of filing: **10.02.83**

(51) Int. Cl.⁴: **C 07 C 51/12, C 07 C 51/56, C 07 C 53/08, C 07 C 53/12, C 07 C 53/122**

(54) Process for the coproduction of carboxylic acids and acid anhydrides.

(30) Priority: **13.02.82 GB 8204284**
**29.04.82 GB 8212527**
**08.05.82 GB 8213382**

(43) Date of publication of application:
**07.09.83 Bulletin 83/36**

(45) Publication of the grant of the patent:
**16.07.86 Bulletin 86/29**

(84) Designated Contracting States:
**BE DE FR GB IT NL**

(56) References cited:
**EP-A-0 046 128**
**EP-A-0 070 787**
**DE-A-1 767 151**
**DE-B-1 966 695**

The file contains technical information submitted after the application was filed and not included in this specification

(73) Proprietor: **BP Chemicals Limited**
**Belgrave House 76 Buckingham Palace Road**
**London, SW1W 0SU (GB)**

(72) Inventor: **Ray, David John Mathieson**
**BP Chemicals Limited Salt End**
**Hedon Hull HU12 8DS (GB)**

(74) Representative: **Crack, Richard David et al**
**c/o The British Petroleum Company plc Patents Division Chertsey Road**
**Sunbury-on-Thames Middlesex, TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

The present invention relates to a process for the coproduction of a carboxylic acid and an acid anhydride especially a lower alkanoic acid, and its corresponding acid anhydride and in particular to a process for the coproduction of acetic acid and acetic anhydride.

Acetic acid and acetic anhydride have been known as industrial chemicals for many years. Acetic anhydride constitutes the second largest end use for acetic acid and is extensively employed in the production of cellulose acetate and other cellulose esters. Smaller quantities are used in the production of aspirin, pharmaceuticals, pesticides and low temperature bleach activators. Acetic acid is used as a preservative and as an intermediate in the production of, for example, acetate esters.

Until recently acetic acid has almost exclusively been produced on an industrial scale either by the oxidation of acetaldehyde or by the oxidation of paraffinic fractions derived from petroleum or natural gas liquids. Now, however, its production by the rhodium catalysed carbonylation of methanol is gaining increasing importance, GB 1233121 (Monsanto) describes and claims a process for the treatment of a reactant which is an alkyl compound having n carbon atoms, where n is a number from 1 to 20 or an aryl compound having n carbon atoms, where n is a whole number from 6 to 20, the said reactant being an alcohol, halide, ester, or ether, to obtain a mixture comprising an organic acid having n+1 carbon atoms, or an ester of the alcohol having n carbon atoms with the said acid, the said process comprising reacting the said reactant at a temperature of at least 50°C with carbon monoxide in the presence as catalyst of rhodium or a rhodium compound and a promoter substance selected from bromine, iodine and bromine and iodine compounds.

On an industrial scale acetic anhydride is currently produced either by reaction of ketene and acetic acid, the ketene being obtained either by dehydration of acetic acid or by decomposition of acetone, or by oxidation of acetaldehyde which also yields acetic acid. Each of these conventional routes has well-known disadvantages which knowledge, together with the increasing attention being paid to chemical syntheses involving carbon monoxide and carbon monoxide/hydrogen mixtures, has led to investigations into the production of acetic anhydride utilising these materials. GB 1468940 (Halcon) describes and claims a process for the preparation of an anhydride of a monocarboxylic acid which comprises reacting a carboxylate ester satisfying the formula RCOOR or an ether satisfying the formula ROR with an acyl halide satisfying the formula RCOX, formed in situ or in a separate stage, under substantially anhydrous conditions, wherein X is iodide or bromide, the Rs may be the same or different and each R is a monovalent hydrocarbyl radical or a substituted monovalent hydrocarbon radical wherein the or each substituent is inert. The acyl halide may be produced by carbonylation of a halide satisfying the formula RX at superatmospheric pressure, R and X being as hereinbefore defined, and the carbonylation may be effected in the presence as catalyst of a Group VIII noble metal, i.e. iridium, osmium, platinum, palladium, rhodium and ruthenium, and optionally a promoter selected from elements having atomic weights greater than 5 of Groups IA, IIA, IIIA, IVB, VIB, the non-noble metals of Groups VIII und the metals of the lanthanide and actinide groups of the Periodic Table, of which suitable metals are lithium, magnesium, calcium, titanium, chromium, iron, nickel and aluminium. US Patent No. 4252741 is one of a number of subsequent improvement patents. This patent relates to an improvement in the carbonylation process of GB 1468940 wherein the volatile products are separated from the catalyst components in a zone in which there is provided a hydrogen partial pressure of at least 10 psi during the separation. In GB 1 523 346 (Hoechst Aktiengesellschaft) a process is disclosed for the preparation of acetic anhydride which comprises treating methyl acetate with carbon monoxide in the presence of a metal selected from ruthenium, rhodium, palladium, osmium, iridium and platinum, hereinafter referred to as a noble metal, in free or combined form and of a halogen in free or combined form. It is stated to be desirable to add to the essential catalyst components further promoters. Such promoters are, for example, complex-forming compounds such as alkyl phosphines or aryl phosphines or even organic nitrogen compounds such as pyridines, pyrollidones, alkyl amines or N-alkyl-derivatives of aniline. Numerous metals or metals compounds, especially carbonyl forming metals such as cobalt, nickel or iron are said to show also a good effect as promoters.

As regards the role of water in the aforesaid carbonylation reactions, it is stated in GB 1233121 that water generally exerts a beneficial effect on the rate of reaction. Adding water, with the feed in excess of the equimolar quantity, e.g. an excess of 50% to 300%, of such equimolar quantity, promotes the production of carboxylic acid, whereas the production of ester is favoured by operation with smaller quantities of water, e.g. 50% to 100% of the equimolar proportion. In the acetic anhydride production process described in GB 1468940, the importance of carrying out the carbonylation reaction under substantially anhydrous conditions, i.e. with essentially dry reactants, is emphasised. In USP 4252741 it is stated that normally the presence of more than 5 mol% of water in any one or more of the reactants should be avoided, the presence of less than 3 mol% of water is desired, and the presence of less than 1 mol% is preferred. In the acetic anhydride production process described in GB 1523346 it is stated that a surprising feature is that the reaction runs speedily under the reaction conditions as per the invention, i.e. also in the absence of any noticeable quantities of water. On the other hand, the starting material may also contain methanol, e.g. up to 25% or minor quantities e.g. not more than 5% initially 1—5% of water, based on the total weight of water+methyl acetate+methanol. In the latter case the process yields not only acetic anhydride, but also acetic acid in a quantity approximately equivalent to the quantity of methanol or water employed.

2

A desirable objective would be to produce in a single plant acetic acid and acetic anhydride in predetermined relative proportions variable over a wide range. Such a plant would be more economic than individual plants in terms of total capital and running costs and would have operational flexibility. We have found that acetic acid and acetic anhydride can be co-produced by co-feeding water to the carbonylation reaction in an amount from 5.5% w/w up to the stoichiometric amount based on the feed, in the presence of one or more Lewis bases. The simultaneous use of Lewis bases or non noble metals as co-promoters can lead to increases in the rate of carbonylation.

Accordingly, the present invention provides a process for the coproduction of a monocarboxylic acid and a monocarboxylic acid anhydride which process comprises reacting a carboxylate ester having the formula $RCOOR^1$ or ether having the formula $ROR^1$, wherein R and $R^1$ are independently monovalent hydrocarbyl radicals, water and optionally an also alcohol at elevated temperature and pressure with carbon monoxide in the presence of a catalyst comprising a noble metal of Group VIII of the Periodic Table according to Mendeleef, a promoter comprising bromine or iodine in free or combined form and a co-promoter comprising a Lewis base or a non noble metal of Group IA, IIA, IIIA, IVB, VIB, or VIII of the Periodic Table or a non-noble metal of Group VB or VIIB capable of forming Lewis bases in solution provided that when R and $R^1$ are methyl, water is added in an amount from 5.5% w/w based on the total weight of water+(ester or ether)+alcohol and when R is other than methyl water is present in an amount from 5.5 mole% in any one or more of the reactants and that the maximum amount of water or equivalent amount of water and alcohol added in either case is 85% of the stoichiometric amount based on the ester or ether.

Suitably the carboxylate ester may be a lower alkyl alkanoate and the ether a lower alkyl ether. Mixtures of esters, mixtures of ethers and mixtures of esters and ethers may be used if so desired. By a suitable choice of esters and/or ethers any desired combination of acids and anhydrides may be produced. For example, methyl acetate and/or dimethyl ether will give acetic acid and acetic anhydride; methyl propionate will give acetic acid, propionic acid, acetic anhydride, propionic anhydride and the mixed acetic/propionic anhydride. Preferably the ester is methyl acetate, and the product is a mixture of acetic acid and acetic anhydride. The preferred ether is dimethyl ether, in which case the product is a mixture of acetic acid and acetic anhydride. The ester or ether may also contain alcohol of formula ROH wherein R is as hereinbefore defined in an amount up to 20% w/w. A very suitable feed is the methanol/methyl acetate binary azeotrope obtained for example from the esterification of methanol with acetic acid. In the case of methyl acetate, the preferred amount of water fed to the reaction is at least 6% (as water) more preferably at least 7% w/w based on the total weight of water+ester+alcohol. At the same time the water content is preferably not greater than 14% w/w or the equivalent amount of water and methanol (3.56 g of methanol being equivalent to 1 g of water) more preferably not greater than 12%. 14% w/w water corresponds to 66% of the stoichiometric amount based on the ester or ether.

The use of pure carbon monoxide is not necessary. It may contain impurities such as carbon dioxide, nitrogen or methane. The carbon monoxide may contain hydrogen or hydrogen can be added separately to the reactor suitably up to 10 vol% to obtain the best compromise between increased reaction rate and formation of hydrogenation products e.g. ethylidene diacetate.

The noble metals of Group VIII of the Periodic Table are iridium, osmium, platinum, palladium, rhodium and ruthenium. Preferably the noble metal is rhodium. The noble metal may be added in any convenient form, for example in the zero valent state or in any higher valent form. Thus the noble metal may be added as the metal itself in finely divided form, as a salt of the metal or as a complex of the metal with ligands, such as carbon monoxide, halides, phosphines and trivalent nitrogen compounds. Suitably the noble metal may be added as a salt of a lower monocarboxylic acid, e.g. rhodium acetate dimer, as a halide, e.g. rhodium chloride or iodide, or as a complex, e.g. $RhCl(PPh_3)_3$ or a rhodium carbonyl. The amount of noble metal added as catalyst may suitably be in the range from 100 to 5000, preferably from 300 to 1500 ppm, based on the noble metal.

The catalyst is promoted by bromine or iodine, preferably iodine, in free or combined form. Suitably the bromine or iodine may be in the form of elementary bromine or iodine, hydrogen bromide or iodide, an inorganic bromide or iodide, such as for example sodium, potassium or cobalt, bromide or iodide, or a quaternary ammonium or phosphonium bromide or iodide, such as for example a tetraalkylammonium bromide or iodide. Preferred are organo-bromides or iodides, such as alkyl bromides or iodides, e.g. methyl iodide, or aryl bromides or iodides. Methyl iodide is particularly preferred. Typically the amount of alkyl bromide or iodide promoter added may be in excess of that required to quaternarise any Lewis base present which is capable of quaternarisation. Typically, the amount of alkyl bromide or iodide added may be in the range from 1 to 30 mol%, preferably from 5 to 15 mol%.

The copromoter can comprise a Lewis base or a non noble metal. Suitable Lewis bases include amines, phosphines and arsines which are capable of forming quaternary salts with the promoter, and a range of non-noble metal compounds which form soluble salts under the reaction conditions. Typical non-noble metal copromoters include metals capable of forming Lewis bases in solution from Groups IVB, VB, VIB, VIIB and VIII of the Periodic Table as published in Lange's Handbook of Chemistry, 12th Edition, Editor J. A. Dean, McGraw-Hill 1979. Further, the copromoter can be a metal described in UK Patent No. 1 468 940 and be an element of Groups IA, IIA, IIIA, IVB, VIBV, Group VIII or a metal of the lanthanide or actinide groups. Specific examples of such metals are chromium, vanadium and zirconium, of which zirconium is preferred.

3

# 0 087 869

The metals may suitably be added in the form of soluble salts, e.g. as zirconyl salts, and the amine or phosphine may suitably be added as such or as a preformed quaternary salt. Suitably the Lewis base is selected from nitrogen heterocycles, e.g. 2-picoline, pyridine, quinoline, pyrrole, pyrrolidine, pyrrolidone, imidazole and N-methyl imidazole, which are capable of forming quaternary ammonium salts under the reaction conditions. Imidazole, for example, is readily alkylated under reaction conditions to N-methyl imidazole, which readily forms quaternary salts soluble under a full range of reaction conditions. The nitrogen heterocyclic compound may suitably be added in an amount up to 10 mol%, preferably from 2 to 10 mol%. The non-noble metal may suitably be added in the range from 0.1 to 2 mol%, preferably from 0.2 to 1 mol%.

The presence of a monocarboxylic acid is desirable because it not only acts as a solvent but also increases the concentrations of essential reactive intermediates. Monocarboxylic acids are a product of the process and will inevitably therefore be present in the reaction but it may be desirable, for example when using low added water concentrations within the aforesaid ranges, to supplement the carboxylic acid produced by the addition of further carboxylic acid. Suitably the added carboxylic acid is identical to the carboxylic acid produced by the process. For example, using methyl acetate or dimethyl ether as the feed it is preferred to add acetic acid. The concentration of carboxylic acid may suitably be in the range from 5 to 50 mol%, preferably from 10 to 35 mol%.

There may also be employed a solvent which is compatible with the catalyst, promoter and copromoters. A preferred solvent is the coproduced acid.

The process may be carried out in the liquid phase or in the vapour phase, preferably in the liquid phase. In the liquid phase, the elevated temperature may suitably be up to 250°C and is preferably in the range from 140 to 200°C. The pressure may suitably be that pressure necessary to prevent carbon monoxide transfer limitation and to keep the reaction mixture in the liquid phase, for example a carbon monoxide partial pressure in the range from 10 to 70 bar. Alternatively, the process may be carried out in the vapour phase, if desired, by appropriate control of the total pressure in relation to the temperature so that the reactants are in the vapour state when contacted with the catalyst. In the case of both liquid and vapour phase operation the catalyst, and optionally also the promoter and/or copromoter(s), may be supported, i.e. they may be dispersed on a conventional support material, such as for example alumina, silica, silica/alumina, zeolites, clays, titania or zirconia. Alternatively the rhodium may be supported on an ion exchange resin or a functionalised inorganic oxide such as those described in the complete specification of GB 1503315 or European patent publication No. 18102 (BP Case 4752). The catalyst and optionally the promoter and/or copromoter(s) may be applied to the support in conventional manner, e.g. by impregnation from solution. The catalyst concentration on the support may suitably be in the range from 0.01 to 10 weight%.

The process of the invention may be carried out batchwise or continuously.

The invention will now be further illustrated by reference to the following Examples and Comparison Tests.

## Example 1

A corrosion resistant autoclave (nominal one litre capacity), fitted with a magnetically driven stirrer unit, was charged with the following mixture:— 2 mol methyl acetate, 2.75 mol acetic acid, 0.5 mol water, sufficient rhodium acetate dimer to give 500 ppm of rhodium in the mixture, 0.2 mol N-methyl imidazole and 0.7 mol methyl iodide, i.e. sufficient to form 0.2 mol N,N-dimethyl imidazolinium iodide under the reaction conditions and still leave an excess of 0.5 mol methyl iodide. The autoclave was then pressurised with 27.6 bar of CO and heated to 175°C, at which point the pressure was further increased to 52.7 bar absolute with CO. The rate of reaction at 175°C was followed by recording the decrease in pressure corresponding to CO consumption. When the pressure had fallen to 49.3 bar, a further 6.9 bar of CO was added, as often as necessary. The maximum rate of reaction corresponded to 393 millibar min$^{-1}$ pressure decrease. Analysis of the product mixture by gas chromatography GC (6.6% w/w PEG 20M on 60—80 mesh Carbopack B column with flame ionisation detector FID) after 60 minutes reaction time showed this to contain 0.83 mol acetic anhydride and 2.35 mol acetic acid derived from methyl acetate. This corresponds to an average CO uptake of 4.8 mol kg$^{-1}$ h$^{-1}$ up to 99% conversion of methyl acetate.

## Comparison test 1

Example 1 was repeated except that no N-methyl imidazole was added and the amount of methyl iodide was reduced to 0.5 mol. The maximum rate of reaction corresponded to 73 millibar min$^{-1}$ CO pressure decrease. This rate is only one fifth of the rate observed in Example 1, i.e. in the presence of the copromoter.

## Examples 2 to 3

Example 1 was repeated except that the level of water was increased to 1.0 mol (Example 2), 1.5 mol (Example 3) and 2.0 mol (Comparison 2), at the same time progressively decreasing the amount of acetic acid to 2 mol to maintain a constant reaction mixture volume. The maximum rates of reaction are given in the following Table 1.

4

TABLE 1

| Example or comparison Test | Water (mol) | Rate of pressure decrease (millibar min$^{-1}$) | Analysis of product after 60 minutes | |
|---|---|---|---|---|
| | | | Acetic anhydride (mol) | |
| 2 | 1.0 | 490 | 0.5 | |
| 3 | 1.5 | 621 | 0.26 | |
| Test 2 | 2.0 | 670 | trace | |

Comparison Tests 3 to 7

Comparison Test 1 was repeated, progressively adding 0.5 mol more water up to a total of 3 mol water. The quantity of acetic acid was also progressively decreased to 2 mol to maintain a constant reaction mixture volume. Maximum rates of reaction increased with water to methyl acetate ratio as shown in the following Table 2.

TABLE 2

| Comparison Test | Water (mol) | Rate of pressure decrease (millibar min$^{-1}$) |
|---|---|---|
| 3 | 1.0 | 221 |
| 4 | 1.5 | 280 |
| 5 | 2.0 | 656 |
| 6 | 2.5 | 759 |
| 7 | 3.0 | 773 |

Analysis by gas chromatography of the product mixture showed acetic acid as the only product from reacted methyl acetate; when all the water present had been used up, reaction effectively ceased.

Comparison of the results in Table 2 with those in Table 1 demonstrates that maximum rates of reaction at 175°C were all higher in the presence of copromoter up to a 1:1 mol ratio of water to methyl acetate.

Example 4

Comparison Test 1 was repeated with the addition of 0.021 mol zirconyl diacetate. The maximum rate of reaction corresponded to a 297 millibar min$^{-1}$ pressure decrease. Analysis of the product mixture after a reaction time of 60 minutes showed the presence of 0.34 mol acetic anhydride and 0.8 mol acetic acid derived from methyl acetate.

Example 5

Comparison Test 2 was repeated along the lines of Example 4 with the addition of 0.021 mol zirconyl diacetate. This time the maximum rate corresponded to 373 millibar min$^{-1}$, giving 0.55 mol acetic anhydride and 2.1 mol acetic acid in 60 minutes.

Examples 6 to 8

The autoclave described above was charged with the following mixtures as shown in Table 3.

5

# 0 087 869

TABLE 3

|  | Example 6 | Example 7 | Example 8 |
|---|---|---|---|
| Methyl acetate, mol | 3.19 | 3.12 | 3.06 |
| Acetic acid, mol | 1.17 | 1.17 | 1.17 |
| Methyl iodide, mol | 0.74 | 0.74 | 0.74 |
| N-methyl imidazole, mol | 0.28 | 0.28 | 0.28 |
| Rhodium acetate dimer, mol ($\times 10^{-3}$) | 3.03 | 3.03 | 3.03 |
| Zirconyl diacetate, mol | 0.021 | 0.021 | 0.21 |
| Water, mol | 0.83 | 1.11 | 1.39 |

The autoclave was, in each case, charged with 32.8 bar of a $CO/H_2$ mixture containing 15.8 volume % $H_2$, heated to a reaction temperature of 185°C, at which point the pressure was further increased to 52.7 bar absolute with CO. Thereafter during reaction the pressure was maintained within +3.5 bar with fresh CO, as necessary. At 80% methyl acetate conversion corresponding to a predetermined cumulative decrease in pressure, the reaction mixture was cooled, discharged and analysed by gas liquid chromatography.

The products shown in Table 4 were observed, derived from methyl acetate:

TABLE 4

| Example | Reaction time minutes | Product mixture | | |
|---|---|---|---|---|
|  |  | Acid anhydride | Acetic acid | Ethylidene diacetate |
| 6 | 29 | 0.95 | 2.17 | 0.20 |
| 7 | 26 | 1.09 | 1.82 | 0.13 |
| 8 | 30 | 0.84 | 2.58 | 0.17 |

Example 9

Example 7 was repeated, at the same total pressure, but without hydrogen present. In this case 1.09 mol acetic anhydride and 2.21 mol acetic acid were obtained from 80% conversion of methyl acetate in 32 minutes. Only 0.03 mol of ethylidene diacetate was observed.

Example 10

A corrosion resistant autoclave (nominal 100 ml capacity) was charged with the following mixture: 0.215 mol methyl propionate, 0.295 mol acetic acid, 0.077 mol methyl iodide, 0.021 mol N-methylimidazole, 0.061 mol water and sufficient rhodium acetate dimer to give 540 ppm of rhodium in solution. The autoclave was then pressurized with 27.6 bar of CO and heated to 175°C, at which point the pressure was brought to 56.9 bar absolute with CO, and subsequently maintained in the range 52 to 56 bar with fresh CO. After 45 minutes the autoclave was cooled, and the product mixture analysed to show the presence of:

| | |
|---|---|
| Acetic acid | 36.3% w/w |
| Propionic acid | 14.9% w/w |
| Acetic anhydride | 10.7% w/w |
| Propionic anhydride | 1.2% w/w |
| Mixed acetic propionic anhydride | 7.7% w/w |

The average CO uptake rate was 4.7 mol $kg^{-1}h^{-1}$ at a methyl propionate conversion of 85%.

6

# 0 087 869

## Claims

1. A process for the coproduction of a monocarboxylic acid and a monocarboxylic acid anhydride which process comprises reacting a carboxylate ester having the formula $RCOOR^1$ or ether having the formula $ROR^1$, wherein R and $R^1$ are independently monovalent hydrocarbyl radicals, water and optionally also an alcohol at elevated temperature and pressure with carbon monoxide in the presence of a catalyst comprising a noble metal of Group VIII of the Periodic Table according to Mendeleef, a promoter comprising bromine or iodine in free or combined form and a copromoter comprising a Lewis base or a non-noble metal of Groups IA, IIA, IVB, VIB or VIII of the Periodic Table or a non-noble metal of Group VB or VIIB capable of forming Lewis bases in solution provided that when R and $R^1$ are methyl, water is added in an amount from 5.5% w/w based on the total weight of water+(ester or ether)+alcohol if present and when R is other than methyl, water is present in an amount from 5.5 mole% in any one or more of the reactants and that the maximum amount of water or equivalent of water and alcohol added in either case is 85% of the stoichiometric amount based on the ester or ether.

2. A process as claimed in claim 1 wherein the ester of the formula $RCOOR^1$ is methyl acetate and the amount of water added is from 6 to 14% w/w or the equivalent amount of water and alcohol and based on the total weight of water+ester+alcohol if present and the product comprises a mixture of acetic anhydride and acetic acid.

3. A process as claimed in claim 2 wherein the amount of water added is from 7 to 12% w/w or the equivalent amount of water and alcohol and based on the total weight of water+ester+alcohol.

4. A process as claimed in any one of claims 1 to 3 wherein the copromoter comprises a nitrogen containing heterocyclic compound.

5. A process as claimed in any one of claims 1 to 3 wherein the copromoter is selected from vanadium, chromium and zirconium.

6. A process as claimed in claim 4 wherein the nitrogen containing heterocyclic compound is selected from 2-picoline, pyridine, quinoline, pyrrole, pyrrolidine, imidazole and N-methyl imidazole.

7. A process as claimed in claim 1 wherein the ester of formula $RCOOR^1$ is methyl propionate and the product comprises propionic anhydride and propionic acid.

8. A process as claimed in any one of claims 1 to 6 wherein hydrogen is added and the product contains ethylidene diacetate.

## Patentansprüche

1. Verfahren zur gemeinsamen Herstellung einer Monocarbonsäure und eines Monocarbonsäureanhydride, wobei das Verfahren die Umsetzung eines Carbonsäureesters mit der Formel $RCOOR^1$ oder eines Ethers mit der Formel $ROR^1$, worin R und $R^1$ unabhängig monovalente Kohlenwasserstoffreste sind, von Wasser und ggf. auch von einem Alkohol bei erhöhter Temperatur und Druck mit Kohlenmonoxid in der Gegenwart eines Katalysators umfaßt, der ein Edelmetall der Gruppe VIII des Periodensystems nach Mendelejeff, einen Beschleuniger, enthaltend Brom oder Jod in freier oder kombinierter Form und einen Co-Beschleuniger enthaltend eine Lewisbase oder ein nicht edles Metall der Gruppen IA, IIA, IVB, VIB oder VIII des Periodensystems oder ein nicht edles Metall der Gruppe VB oder VIIB, des zur Bildung von Lewisbasen in Lösung fähig ist, umfaßt, vorausgesetzt, daß wenn R und $R^1$ Methyl sind, Wasser in einer Menge von 5.5% (Gewicht/Gewicht) bezogen auf das Gesamtgewicht von Wasser+(Ester oder Ether)+Alkohol, wenn vorhanden, zugegeben wird, und wenn R etwas anderes als Methyl ist, das Wasser in einer Menge von 5,5 Mol% in irgendeinem oder mehreren der Reaktanten vorhanden ist, und daß die maximale zugegebene Menge von Wasser oder von Äquivalent von Wasser und Alkohol in jedem Fall 85% der stöchiometrischen Menge bezogen auf den Ester oder Ether ist.

2. Verfahren nach Anspruch 1, worin der Ester der Formel $RCOOR^1$ Methylacetat ist und die zugegebene Menge an Wasser von 6 bis 14% (Gewicht/Gewicht) oder die äquivalente Menge von Wasser und Alkohol, bezogen auf das Gesamtgewicht von Wasser+Ester+Alkohol, wenn vorhanden, ist, und wobei das Produkt eine Mischung von Acetanhydrid und Essigsäure umfaßt.

3. Verfahren nach Anspruch 2, worin die zugegebene Menge des Wassers 7 bis 12% (Gewicht/Gewicht) oder die äquivalente Menge von Wasser und Alkohol, bezogen auf das Gesamtgewicht von Wasser+Ester+Alkohol ist.

4. Verfahren nach einem der Ansprüche 1 bis 3, worin der Co-Beschleuniger eine stickstoffhaltige heterocyclische Verbindung umfaßt.

5. Verfahren nach einem der Ansprüche 1 bis 3, worin der Co-Beschleuniger ausgewählt wird unter Vanadium, Chrom und Zirkonium.

6. Verfahren nach Anspruch 4, worin die stickstoffhaltige heterocyclische Verbindung ausgewählt wird unter 2-Pikolin, Pyridin, Chinolin, Pyrrol, Pyrrolidin, Imidazol und N-Methyl-Imidazol.

7. Verfahren nach Anspruch 1, worin der Ester der Formel $RCOOR^1$ Methylpropionat ist und das Produkt Propionsäureanhydrid und Propionsäure umfaßt.

8. Verfahren nach einem Ansprüche 1 bis 6, worin Wasserstoff zugegeben wird und das Produkt Ethylidendiacetat enthält.

**Revendications**

1. Procédé pour la production simultanée d'un acide monocarboxylique et d'un anhydride d'acide monocarboxylique, ce procédé comprenant lu réaction d'un ester carboxylique, de formule $RCOOR^1$, ou d'un éther de formule $ROR^1$, dans lesquelles R et $R^1$ sont, indépendamment, des radicaux hydrocarbyles monovalents, de l'eau et éventuellement aussi d'un alcool, à température et pression élevées, avec le monoxyde de carbone en présence d'un catalyseur comprenant un métal noble du groupe VIII du Tableau Périodique selon Mendeleef, un promoteur comprenant du brome ou de l'iode, sous forme libre ou combinée, et un copromoteur comprenant une base de Lewis ou un métal non noble des groupes IA, IIA, IVB, VIB ou VIII du Tableau Périodique ou un métal non noble du groupe VB ou VIIB capable de former des bases de Lewis en solution, à la condition que, lorsque R et $R^1$ représentent chacun un groupe méthyle, on ajoute de l'eau en une quantité de 5,5% en poids/poids sur la base du poids total de l'eau+(ester ou éther)+alcool s'il est présent, et que, lorsque R représente autre chose qu'un groupe méthyle, l'eau soit présente en une quantité de 5,5 moles% dans n'importe lequel des corps destinés à réagir ou dans plusieurs de ces corps, et que la quantité maximale d'eau ou d'équivalent d'eau et d'alcool que l'on ajoute dans l'un ou l'autre cas, représente 85% de la quantité stoéchiométrique sur la base de l'ester ou de l'éther.

2. Procédé selon la revendication 1, dans lequel l'ester de formule $RCOOR^1$ est l'acétate de méthyle, et la quantité d'eau ajoutée est de 6 à 14% en poids/poids ou la quantité équivalente d'eau et d'alcool et elle se fonde sur le poids total de l'eau+ester+alcool s'il y en a, et le produit comprend un mélange de l'anhydride acétique et de l'acide acétique.

3. Procédé selon la revendication 2, dans lequel la quantité d'eau ajoutée est de 7 à 12% en poids/poids ou la quantité équivalente d'eau et d'alcool et sur la base du poids total de l'eau+ester+alcool.

4. Procédé selon l'une quelconque des revendications 1 à 3, dans lequel le groupe promoteur est un composé hétérocyclique contenant de l'azote.

5. Procédé selon l'une quelconque des revendications 1 à 3, dans lequel le groupe promoteur est choisi parmi le vanadium, le chrome et le zirconium.

6. Procédé selon la revendication 4, dans lequel le composé hétérocyclique contenant de l'azote est choisi parmi la 2-picoline, la pyridine, la quinoléine, le pyrrole, la pyrrolidine, l'imidazole et le N-méthyl imidazole.

7. Procédé selon la revendication 1, dans lequel l'ester de formule $RCOOR^1$ est le propionate de méthyle, et le produit comprend de l'anhydride propionique et de l'acide propionique.

8. Procédé selon l'une quelconque des revendications 1 à 6, dans lequel on ajoute de l'hydrogène et le produit contient du diacétate d'éthylidène.